# EUROPEAN PATENT SPECIFICATION

(11) **EP 0 783 276 B1**
(45) Date of publication and mention of the grant of the patent: **07.08.2002**
(21) Application number: 95942266.8
(22) Date of filing: 22.12.1995
(51) Int. Cl.: A61B 17/64

(54) **ARTICULATION SUPPORT DEVICE**
GELENKSTÜTZVORRICHTUNG
DISPOSITIF DE SOUTIEN DES ARTICULATIONS

(30) Priority: 23.12.1994 IT MI942615
(43) Date of publication of application: 16.07.1997
(73) Proprietor: Danieli, Guido A., 87030 Arcavacata di Rende (IT); Danieli, Giorgio S., 20122 Milano (IT)
(72) Inventor: Danieli, Guido A., 87030 Arcavacata di Rende (IT); Danieli, Giorgio S., 20122 Milano (IT)
(86) International application number: IT9500225
(87) International publication number: WO9619944

(56) References cited:
- EP-A- 0 460 944
- WO-A-88/03395
- WO-A-92/02184
- GB-A- 2 001 533
- GB-A- 2 026 321
- CLINICAL BIOMECHANICS, vol. 9, no. 1, 1 January 1994, pages 51-59, XP000418367 GARDNER ET AL.: "THREE-DIMENSIONAL MOVEMENT AT EXTERNALLY FIXATED TIBIAL FRACTURES AND OSTEOTOMIES DURING NORMAL PATIENT FUNCTION"

## Description

### Technical Field

In case of articulation's trauma, the usual procedure is to block the articulations till healing. This may cause a permanent articulation damage and, in the best condition, a long period of rehabilitation. Alternatively, donjoys or elements of plaster of Paris connected by hinges may be used. The present invention deals with a system able to allow motion of physiological articulations in absence of load, being the weight supported by an external device, connected through pins to the patient's bone structure.

### Background Art

Currently the task of supporting a damaged articulation when motion is to be permitted is performed by donjoys or by the aforementioned plaster of Paris elements. These however cannot fully support the articulation because they are not connected to the bone structure. On the other hand external articulation fixators that are not guided during calibration by a computer may cause more problems than they solve.

### Disclosure of Invention

In order to solve the problem of supporting the weight while allowing at least one degree of motion, a system is needed in which it is first possible to measure the individual patient's articulation geometry, rigidly connecting the proximal and the distal bones facing the articulation, with a measuring device, able to supply a computer the instantaneous values of six parameters used to fully describe the relative motion between its extremes.

Thus, in the case of the knee, pins are inserted both in the femur and tibia. The first member of the measuring device is connected to the femoral pins, while the last is clamped to the tibia pins, and then the knee is articulated while proceeding with the measurement.

It is obvious that in the case of an articulation damaged by a trauma, first the doctor should operate the patient to reconstruct the articulation, then, he should move the patient's articulation allowing via the measuring device to detect the correct patient's geometry.

Then the mechanism that best fits the individual patient, never allowing compression of the articulation to be healed, is to be determined via dedicated software, together with its position with respect to the pins inserted by the doctor into the patient's skeletal structure.

As a third step the mechanism determined as suitable for the patient, should be correctly positioned, placing its frame at the right place with respect to the proximal pins, and similarly its rod, with respect to the distal ones.

To this end each of the two portions of the fixator joining the bone to the articulating mechanism have to be supplied with six degrees of freedom between the extremes. In between, in the case of a knee, a four bar linkage may be inserted whose geometry is fixed by the length of the four bars and by the initial and final angle between the frame (the proximal part of the fixator) and the first crank linking to the rod. In other cases, a simple hinge, but correctly placed, may be sufficient.

The adjustment of the fixator is then performed by comparing, with the help of the measuring device, the recorded individual motion with the fixator's motion, and separately regulating the correct position for each of the six degrees of freedom of the fixator. This allows minimising errors in positioning by using a string of data (the data obtained measuring the motion of the rod) rather than a single one (the position of the frame), more sensitive to digitisation errors.

At the end of this procedure the patient will be able to move the articulation being supported by a purely mechanical system, not needing further adjustments.

Notice that the system described is applicable to any articulation, and not only to the knee, even if the prototypes of the system described were developed only for this.

Also notice that six degrees of freedom necessary for each of the two portions of the fixator may belong to the fixator itself, or may be part of a different device, active (driven by the computer) or passive (driven by the operator), that should be used as a "fixation mask" to guide mounting of the real fixator while respecting the required geometry.

Such fixator, that may be made much more freely, has the only task to withstand the forces while keeping the frame of the selected mechanism at the right position with respect to the pins on the proximal side of the articulation, and the rod of said mechanism at the right position with respect to the pins on the distal side of the articulation.

### Brief Description of the Drawings.

Table 1 shows a possible execution of an articulation fixator for the knee, on which a, b and c are the roto-traslational axes that supply the six degrees of freedom needed on the proximal structure. On the same table, d, e and f indicate the other six degrees of rototranslation relative to the distal structure. In between a four bar linkage is inserted. Also notice that the translational and rotational adjustment of each couple of degrees of freedom can be performed, for example, using counter nuts.

Section AA on the same table shows as, in order to uniquely position the clamp with respect to the bone patient, one of the slots on the clamp needs to present a reference hole, to be fitted by a reference pin.

Still worth of notice in the same figure the fact that one of the constraints **ced** (the ones relative to the translational component) are to be set freely by the doctor, since they allow to move the four link bars along planes parallel to motion.

In particular, assuming to utilise a double four link bar for stability, constraints **ced** allow positioning the four link bars on both sides of the articulation.

Also notice that the system shown in figure is only one of the infinite possible systems bearing six degrees of freedom.

For instance, in the measuring device used in the research that led to the present Patent Application (Table 2), the six degrees of freedom are all rotational, but allow as well placing the two bodies in any reciprocal position. Similarly the six degrees of freedom of both the measuring device and the two fixator's portions can be set as described in US Patent n° 5,152,280 (Table 3), or in any other configuration, as long as at least three rotational degrees are left.

Table 4 shows the distal portion of the measuring device clamped on one side to the proximal pins, and on the other to the rod of the four link bar, for adjustment of the six degrees of freedom of the proximal portion of the fixator, while Table 5 shows the measuring device hooked between the fixator end clamps, in order to set the values of the six last degrees of freedom.

As alternative to the seventeen degrees of freedom fixator one can use a six degrees of freedom system, thereby called the fixation mask, as the one shown in Table 6. In this case the system itself is positioned coupling the six degrees of freedom fixation mask to the measuring device between the clamp and the rod of the mechanism, used to reproduce the articulation movement, similarly to what shown in Table 4, then. through the computer controlled measuring device, one proceeds to the measurement of the trajectories described by the rod of the articulating mechanism.

Once the correct configuration has been obtained through sequential adjustment and locking of the six constraints (the degrees of freedom of the fixation mask), one can mount, using the mask as a guide, the structure of the real fixator.

At this point the fixation mask is removed and connected between the frame of the mechanism and the end clamp on the distal side, while the measuring device is connected between end clamps, setting this time the last six degrees of freedom on the proximal side. This is shown in Table 7.

Clearly it is also possible to use as a fixation mask a complete fixator as per Table 4.

As further alternative (Table 8), one can use a six degrees of freedom active positioning device (mounting robot) using it as substitution to the fixation mask. The active device, under computer control, will move its extremes to assume the correct relative position, allowing, as in the aforementioned case, the assembly of an external structure. In particular the fixator's extremes will have to be connected one on constraint (a) and one on constraint (f) of table 7, whereas (a), (b), and (c) are active rotating devices, while the remaining three supply the translational degrees of freedom.

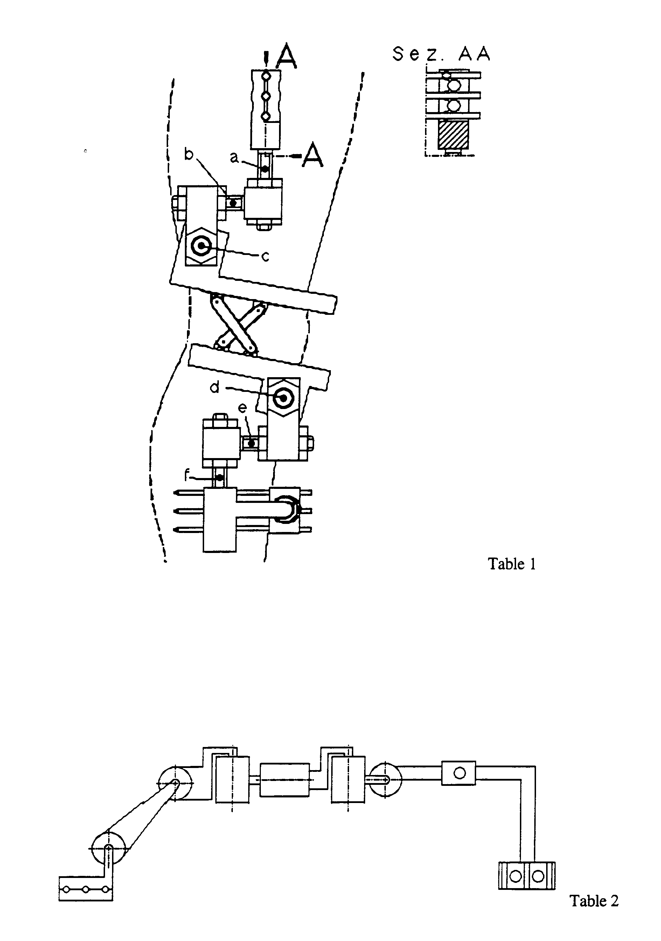

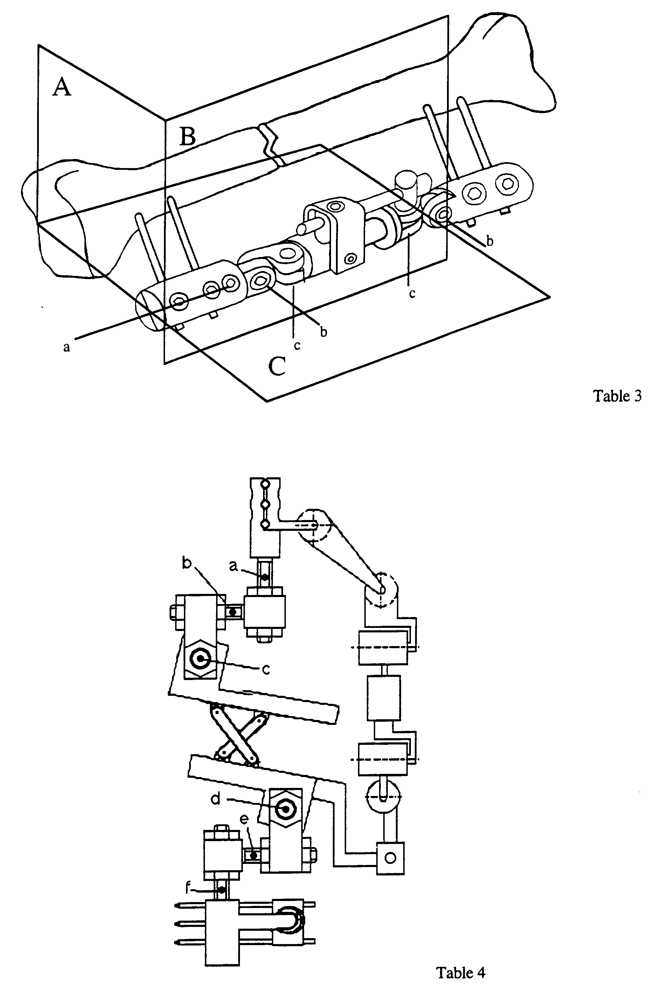

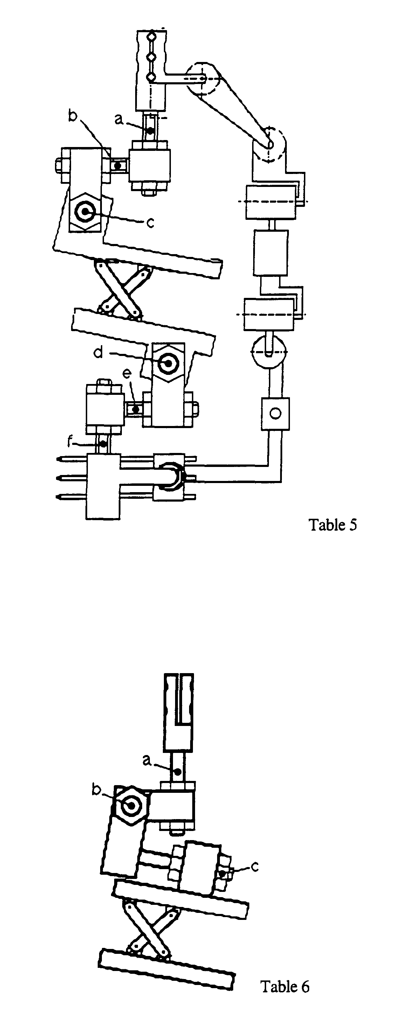

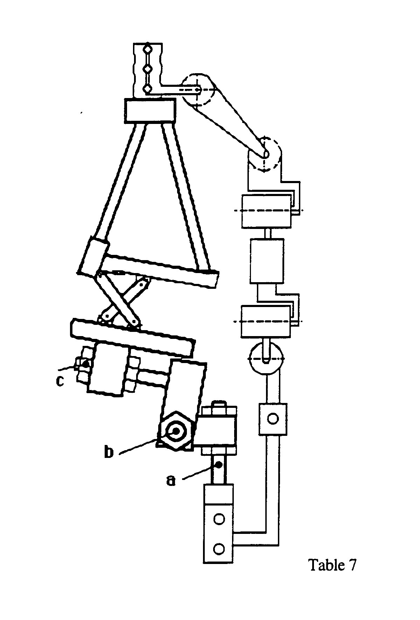

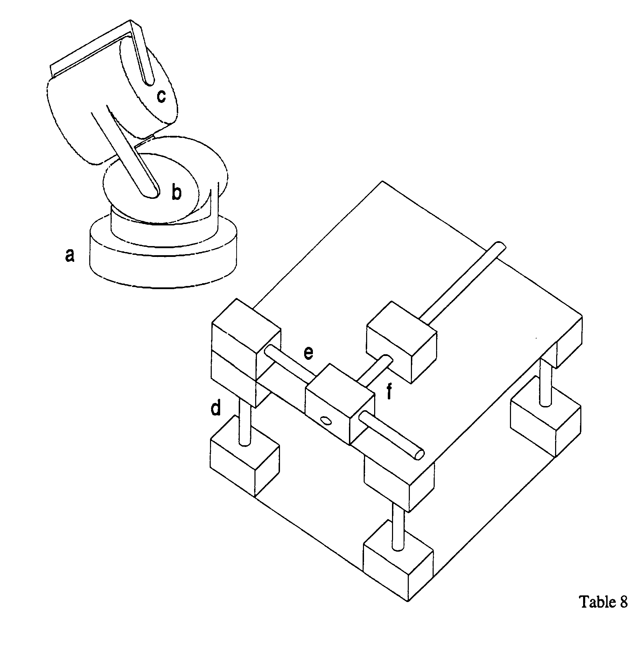

## Claims

1. Fixation system for articulations leaving one or two guided degrees of motion, comprising a device for external support of articulations, defined from now on the "external fixator", and a measuring device allowing to record the instantaneous values of six parameters fully describing the relative motion between the bones facing an articulation, said measuring device being coupled to an electronic instrument able to detect and elaborate data,
the external fixator being composed of three main elements, a proximal member having a proximal clamp holding pins adapted to be inserted into the patient's bone structure, a distal member having a distal clamp also holding pins adapted to be inserted into the patient's bone structure, but on the other side of the articulation, and a "mechanism", the third main element, positionned between said proximal member and said distal member, said mechanism leaving one or two degrees of freedom, the positioning of said mechanism being selectable via software to correctly reproduce the individual patient's kinematism as measured by the measuring device,
both said proximal member and said distal member being a six independent degrees of freedom system, which can be individually set, in order to allow the correct positioning of the mechanism with respect to the pins inserted into the patient's bones,
the clamps each being provided with a reference slot in order to allow uniquely positioning both the measuring device and the fixator with respect to the patient's bony structure.

2. Fixation system for articulations leaving one or two guided degrees of freedom according to claim 1, in which the electronic instrument able to detect and elaborate data consists in a computer with data acquisition board and special purpose software.

3. Fixation system for articulations leaving one or two guided degrees of freedom according to claim 1, in which the mechanism controlling the motion consists in a four bars linkage or a hinge.

## Patentansprüche

1. Fixateursystem für Gelenke, das einen oder zwei geführte Bewegungsfreiheitsgrade hat, umfassend eine Vorrichtung für die äußere Gelenkunterstützung, nachstehend "äußerer Fixateur" genannt, und eine Meßeinrichtung für die Aufnahme der momentanen Werte von sechs Parametern, welche die Relativbewegung zwischen den zu einem Gelenk gehörenden Knochen vollständig beschreiben, welche Meßeinrichtung mit einem elektronischen Instrument zur Erfassung und Verarbeiten der Daten verbunden ist, wobei der äußere Fixateur aus drei Hauptelementen zusammengesetzt ist, nämlich einem proximalen Glied mit einem proximalen Klemmorgan zum Halten von Stiften, die in die Knochenstruktur des Patienten einzusetzen sind, einem distalen Glied mit einem distalen Klemmorgan, das ebenfalls Stifte trägt, die in die Knochenstruktur des Patienten von der gegenüberliegenden Seite des Gelenkes einzusetzen sind, sowie einem "Mechanismus", dem dritten Hauptelement, der zwischen dem proximalen Glied und dem distalen Glied angeordnet ist und ein oder zwei Freiheitsgrade hat und dessen Lage durch Software wählbar ist, um den individuellen, von der Meßeinrichtung ermittelten Bewegungsablauf des Patienten exakt zu reproduzieren, wobei sowohl das proximale Glied als auch das distale Glied ein System von sechs unabhängigen Freiheitsgraden bilden, das individuell einstellbar ist, um die korrekte Positionierung des Mechanismus bezüglich der in die Knochen des Patienten eingesetzten Stifte zu gewährleisten, wobei jedes Klemmorgan einen Bezugsschlitz aufweist, über den sowohl die Meßeinrichtung als auch der Fixateur relativ zur Knochenstruktur des Patienten eindeutig positioniert werden kann.

2. Fixateursystem mit einem oder zwei geführten Bewegungsfreiheitsgraden für Gelenke nach Anspruch 1, bei dem das elektronische Instrument zur Erfassung und Auswertung der Daten aus einem Computer mit einer Datenerfassungsschaltung und einer Spezialsoftware besteht.

3. Pixateursyetem mit einem oder zwei geführten Bewegungsfreiheitgraden für Gelenke nach Anspruch 1, bei dem der Mechanismus zur Bewegungssteuerung aus einem Viergelenkgetriebe oder einem Scharnier besteht.

## Revendications

1. Système de fixation pour articulation qui laisse un ou deux degrés guidés de mouvement, comprenant un appareil pour support externe d'articulations, et défini, de ce moment, le "fixateur externe", et un appareil de mesure permettant l'enregistrement des valeurs instantanées de six paramètres décrivant parfaitement le mouvement relatif aux os qui sont unis par une articulation, ledit appareil de mesure étant associé à un instrument électronique capable de détecter et de faire des calculs sur des données, le "fixateur externe" étant composé de trois éléments principaux, un membre proximal doté d'une pince proximale, pour tenir des épingles adaptées pour être insérées dans la structure de l'os du malade, un membre distal ayant une pince distale qui a aussi des épingles adaptées pour être insérées dans la structure de l'os du malade, mais sur l'autre côté de l'articulation, et un "mécanisme", le troisième élément principal, placé entre ledit membre proximal et ledit membre distal, ledit mécanisme qui laisse un ou deux degrés de liberté, la position du dit mécanisme étant sélectionnable par logiciel pour reproduire le cinématisme individuel du malade comme correctement mesurée par l'appareil de mesure, étant ledit membre proximal et ledit membre distal, tous les deux un système à six degrés indépendants de liberté, qui peut être individuellement réglé pour permettre le positionnement correct du mécanisme en ce qui concerne les épingles insérées dans les os du malade, étant chaque pince fournie d'une fente de référence pour permettre le placement de l'appareil de mesure et du fixateur avec référence à la structure osseuse du malade dans une manière unique.

2. Système de fixation des articulations qui laisse un ou deux degrés de liberté selon la revendication 1, dans la quelle l'instrument électronique capable de mesurer et élaborer des données est un ordinateur avec un circuit d'acquisition des données et un software destiné à cela.

3. Système de fixation des articulations qui laisse un ou deux degrés de liberté selon la revendication 1, dans la quelle le mécanisme qui contrôle le mouvement est un mécanisme à quatre barres ou une charnière.
